# EUROPÄISCHE PATENTSCHRIFT

(11) **EP 0 352 761 B1**
(45) Veröffentlichungstag und Bekanntmachung des Hinweises auf die Patenterteilung: **04.10.1995**
(21) Anmeldenummer: 89113741.6
(22) Anmeldetag: 25.07.1989
(51) Int. Cl.: C12P 21/08, C07K 17/02, A61K 38/00, C12N 15/12

(54) **Antigenkonstrukte von "Major Histocompatibility Complex" Klasse I Antigenen mit spezifischen Trägermolekülen, ihre Herstellung und Verwendung**
Antigens composed of "major histocompatibility complex" class I antigens and specific carrier molecules, their production and use
Antigènes composés d'antigènes classe I du "major histocompatibility complex", leur production et utilisation

(30) Priorität: 28.07.1988 DE 3825615
(43) Veröffentlichungstag der Anmeldung: 31.01.1990
(73) Patentinhaber: BEHRINGWERKE Aktiengesellschaft, 35001 Marburg (DE)
(72) Erfinder: Seemann, Gerhard, Dr., D-3550 Marburg (DE); Bosslet, Klaus, Dr., D-3550 Marburg (DE); Sedlacek, Hans Harald, Dr., D-3550 Marburg (DE)

(56) Entgegenhaltungen:
- EP-A- 0 141 079
- EP-A- 0 213 581
- EP-A- 0 226 069
- HUMAN IMMUNOLOGY, Band 17, Nr. 3, 1986, Seiten 246-249; J.J. VAN ROOD: "HLA antigens as carrier molecules"

## Beschreibung

Die Erfindung betrifft Antigenkonstrukte, die aus der Verknüpfung von "Major Histocompatibility Complex" (MHC) Klasse I Antigenen mit spezifischen Trägermolekülen resultieren.

Gewebeabstoßungsreaktionen sind die stärksten T-Zell-vermittelten Immunreaktionen, die bekannt sind. Bei Individuen derselben Spezies werden sie durch allogene Unterschiede der Klasse I und Klasse II MHC Antigene verursacht. Bei Organübertragungen beispielsweise werden die gegebenenfalls vorhandenen allogenen Determinanten der MHC Antigene des Spendergewebes von allospezifischen T-Zellen des Empfängers als fremd erkannt, eine T-Zell Immunantwort wird induziert und es kommt zur Abstoßungsreaktion, sofern keine immunsuppressive Therapie eingeleitet wurde bzw. eine solche nicht mehr ausreicht.

Ferner ist bekannt, daß MHC Klasse I Antigene Glykoproteine sind, die auf der Oberfläche aller kernhaltigen Zellen ausgeprägt werden. Sie bestehen aus einer schweren Kette, die von MHC Klasse I Genen kodiert wird, und einer leichten Kette, dem β₂-Microglobulin, das nicht-kovalent mit der schweren Kette assoziiert ist. Der extrazelluläre Teil der schweren Kette ist in drei Domänen gefaltet, von denen die ersten beiden Domänen (alpha₁ und alpha₂) beim Vergleich der Aminosäuresequenzen bislang bekannter Klasse I MHC Antigene verschiedener Individuen einen ausgeprägten Polymorphismus aufweisen. Sie dienen der Antigenpräsentation und tragen die allogenen Determinanten. Die dritte extrazelluläre Domäne zeigt eine konserviertere Sequenz. Die Assoziation mit β₂-Microglobulin ist für eine korrekte Faltung der schweren Kette und für den Transport des Moleküls auf die Zelloberfläche essentiell.

Die Isolation und Charakterisierung mutierter MHC Klasse I Antigene bei der Maus zeigte, daß für die Induktion einer Abstoßungsreaktion bereits wenige Aminosäureunterschiede auf der alpha₁ und alpha₂ Domäne zwischen Spender und Empfänger genügen (Nathenson et al., Ann. Rev. Immunol.,1986, 4, 471-502). Auch beim Menschen wurde gezeigt, daß geringe Unterschiede zwischen Spender und Empfänger zur Abstoßung eines Transplantates führen (Dausset, J., Rapaport, F.T., Legrand, L., Colombani, J., Marcelli-Barge, A.: Skin allograft survival in 238 human subjects: Role of specific relationships at the four gene sites of the first and the second HL-A loci., Histocompatibility Testing (1970) S. 381-397, Terasaki P.L. (Ed.)). Aus dem vorstehend Gesagten stellte sich die Aufgabe, die spezifische Induzierbarkeit und Stärke der zellulären Immunantwort bei der Gewebeabstoßungsreaktion zu nutzen, um ausgewählte Zielzellen zu schädigen oder zu zerstören.

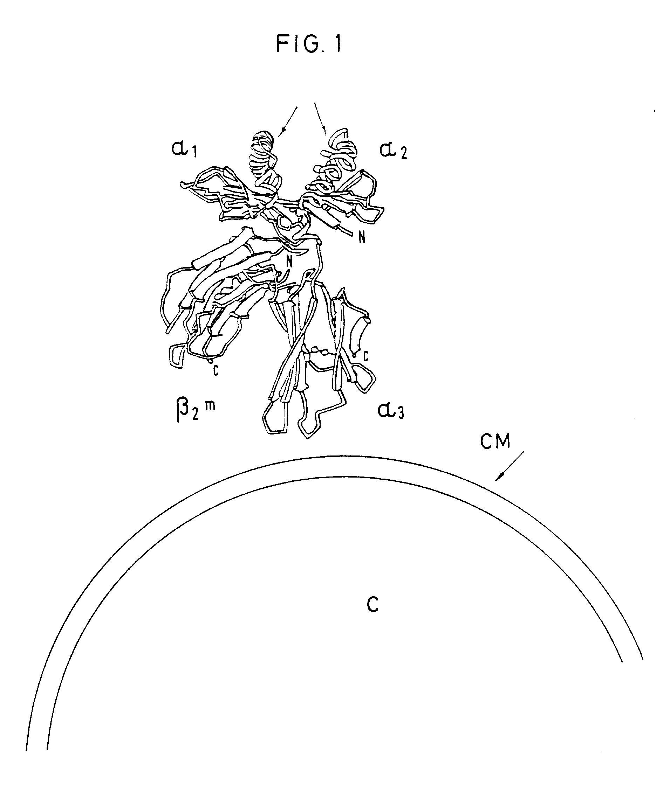

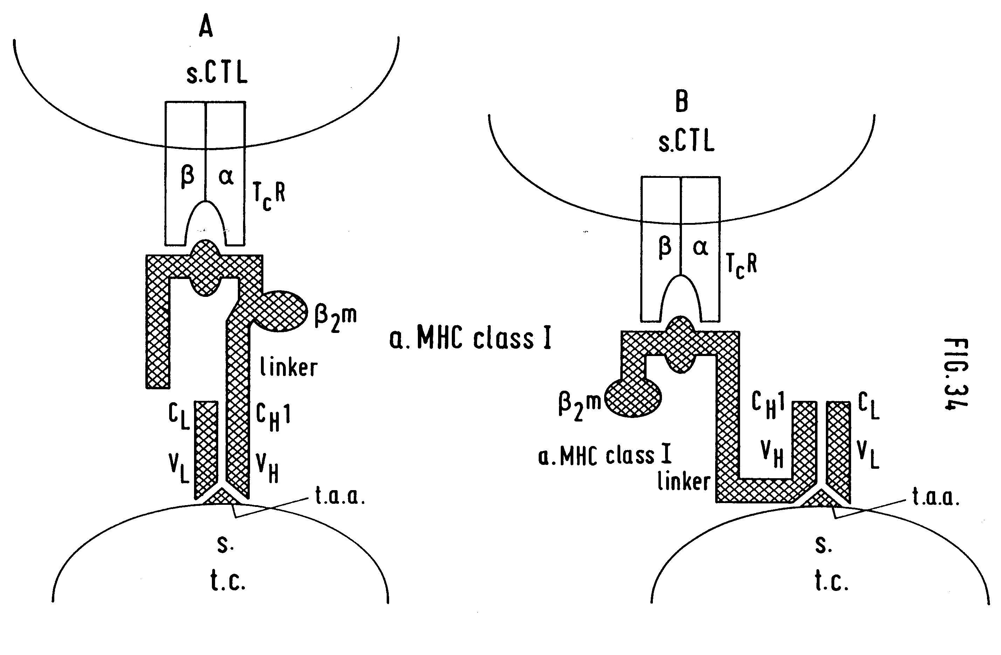

Es wurde gefunden, daß sich Zielzell-spezifische Träger, z.B. vorzugsweise monoklonale Antikörper (mAK), aber auch polyklonale Antikörper oder an zellständige Rezeptoren bindende Moleküle kovalent an das N- oder C-terminale Ende eines allogenen MHC Klasse I Moleküls koppeln lassen, ohne dadurch die allogenen Determinanten nachteilig zu verändern. Mit Hilfe dieses Zielzell-spezifischen Trägers wird das MHC Klasse I Molekül spezifisch auf die Zielzellen gebracht, was zur Aktivierung allospezifischer T-Zellen und damit zur Zerstörung der Zielzellen durch allospezifische zytotoxische T-Zellen führt. Eine Erklärung für das Gelingen der Kopplung eines Zielzell-spezifischen Trägers an das N- oder C-terminale Ende eines MHC Klasse I Moleküls unter Erhalt der allogenen Determinanten liegt darin, daß das N-terminale Ende des MHC Klasse I Moleküls auf der zur Zelle gerichteten Seite der alpha₁- und alpha₂-Domänen liegt, während sich die allogenen Determinanten auf der von der Zelle abgewandten Seite der alpha₁- und alpha₂- Domänen befinden (Fig. 1, Fig. 34).

Aufgrund des großen Polymorphismus der MHC Klasse I Antigene in der menschlichen Bevölkerung gelingt es, mit Hilfe von nur zwei verschiedenen ausgewählten MHC Klasse I Molekülen, z.B. HLA B27w und HLA B27k bei nahezu 100% der Population eine Abstoßungsreaktion zu induzieren. HLA B27w und HLA B27k sind zwei durch zytotoxische T-Lymphozyten definierte Subtypen der serologisch definierten HLA B27 Spezifität. In der kaukasoiden Bevölkerung prägen ca. 7% der Individuen HLA B27w und ca. 1% HLA B27k aus. Durch die Verwendung beispielsweise beider HLA B27 Subtypen zur Allogenisierung entsprechend dieser Erfindung können nahezu 100% der kaukasoiden Population behandelt werden. Man kann jedoch gemäß der Erfindung jedes beliebige MHC-Klasse I Antigen kovalent an die betreffenden spezifischen Träger koppeln, falls o.g. Antigene im betreffenden Empfänger nicht zur Aktivierung allospezifischer T-Zellen und anschließender Schädigung bzw. Zerstörung der Zielzellen führen. Als Zielzellen können im Körper nicht gewünschte und/oder krankmachende Zellen wie z.B. Tumorzellen gelten. Die erfindungsgemäßen Antigenkonstrukte sind demzufolge geeignet für die Tumortherapie. Jedoch auch andere Erkrankungen, die durch Zellen bzw. deren Produkte verursacht sind und durch Eliminierung dieser Zellen günstig beeinflußt werden, können mit den erfindungsgemäßen MHC Klasse I Antigenkonstrukten therapiert werden. Die Wirkungsweise der in den Beispielgruppen I und II beschriebenen Hybridmolekülen beruht darauf, daß diese aufgrund der Spezifität des Antikörperanteils an ein zellständiges Antigen binden können. Durch den HLA B27 Anteil des Fusionsmoleküls wird die Oberfläche der Zielzelle mit einem allogenen MHC Klasse I Molekül maskiert. Diese allogenen Klasse I Moleküle können dann von syngenen, allospezifischen, zytotoxischen T-Zellen erkannt werden, was zur Zerstörung der Zielzellen durch die allospezifischen zytotoxischen T-Zellen führt. Die Erfindung betrifft demgemäß
a) MHC Klasse I Antigene, die N- oder C-terminal mit spezifischen Trägern verknüpft sind, wobei die Verknüpfung kovalent bewirkt wird und wobei die spezifischen Träger selektiv an Zielzellen binden, sie vorzugsweise monoklonale, aber auch polyklonale Antikörper bedeuten, sie jedoch ganz allgemein Rezeptor-bindende Moleküle sind, die an die jeweiligen Zellrezeptoren binden,
b) Verfahren zur Herstellung der MHC Klasse I Antigenkonstrukte, und
c) die Verwendung der unter a) und b) genannten MHC Klasse I Antigenkonstrukte zur Herstellung eines Arzneimittels zur Schädigung oder Eliminierung von Zielzellen.

Die Erfindung ist ferner in den nachfolgenden Beispielen und den Patentansprüchen beschrieben, wobei sie aber nicht darauf beschränkt werden darf.

Die nachstehend aufgeführten Beispiele 1 - 17 beschreiben ein erfindungsgemäßes Konstrukt aus dem Nitrophenol (NP-) spezifischen Maus mAK B/1-8 V_{H}-Gen (1), einem humanen IgG C-F (ab′)₂ Gen (2) und einem HLA B27w Gen (3). (1) und (2) sind dabei beispielhaft für den spezifischen Trägeranteil - hier ein mAK gegen NP - zu sehen, während (3) für ein HLA Klasse I Antigen steht.

Das o.g. Konstrukt wird nach entsprechender Transformation von solchen Myelomzellen ausgeprägt und sezerniert, die ein humanes β₂-Microglobulin und eine leichte Kette eines Immunglobulins enthalten, deren V-Gen mit V_{H} B/1-8 eine NP-Bindungsstelle bildet, wie etwa die Maus-Myelomzelle J 558 L (Oi, V.T., Morrison, S.L., Herzenberg, L.A.,Berg, P.: Immunoglobulin gene expression in transformed lymphoid cells. Proc. Natl. Acad. Sci. USA 80, 825, 1983). Durch Austausch des V_{H} Gens der schweren Kette und Verwendung einer entsprechenden leichten Kette kann das mAK/HLA B27w Fusionsprodukt mit jeder gewünschten Spezifität versehen werden, für die ein spezifischer oder selektiver mAK existiert.

### Beispiele

### I Die Beispiele 1 bis 13 zeigen die Konstruktion eines HLA B27/MAK Fusionsgens mit dem HLA B27 Anteil am 3′ Ende des monoklonalen Antikörpers

### A) Vorbereitung des mAK C-Gen Anteils (IgG₃ C-Gen)

### Beispiel 1

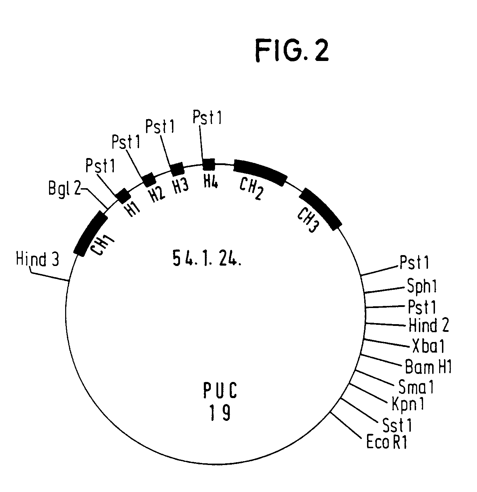

Ein humanes IgG₃ C-Gen wurde aus einer humanen Genbank in EMBL3 Phagen isoliert (Frischauf, A.-M., Lehrach, H., Proustka, A., Murray, N.: Lambda replacement vectors carrying polylinker sequences. J. Mol. Biol. 170, 827-842 (1983) und Seemann, G.H.A., Rein, R.S., Brown, C.S., Ploegh, H.L.: Gene conversion-like mechanisms may generate polymorphism in human class I genes. The EMBO Journal 5, 547-552 (1986)) und als 3,1 kb großes HindIII/SPH I Fragment in den Plasmidvektor pUC 19 subkloniert (Klon 54.1.24) (Fig. 2).

Alle in diesen und in den folgenden Beispielen verwendeten Techniken wurden, wenn nicht anders angezeigt, aus Maniatis, T., Fritsch, E.F., Lehrach, H. und Frischauf, A.-M. Laboratory Manual EMBL (1982), Heidelberg; Sambrook, J.: Molecular Cloning: A laboratory manual (1982), Cold Spring Harbour Laboratory, entnommen.

### Beispiel 2

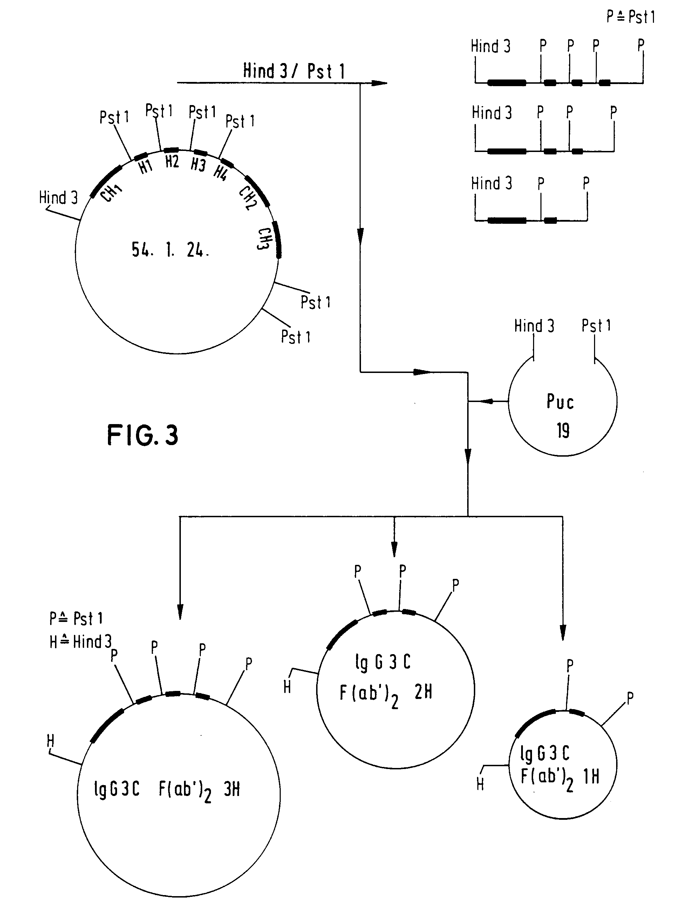

Der 54.1.24 Klon wurde einem vollständigen HindIII und einer partiellen Pst1 Restriktionsverdauung unterzogen. Dabei entstehen unter anderem Restriktionsfragmente, die das C_{H1} Exon und ein, zwei oder drei "Hinge-Exons" enthalten. Diese Fragmente wurden aus einem Agarosegel ausgeschnitten und in einen mit HindIII und Pst1 geschnittenen pUC19 Vektor kloniert (Fig. 3).

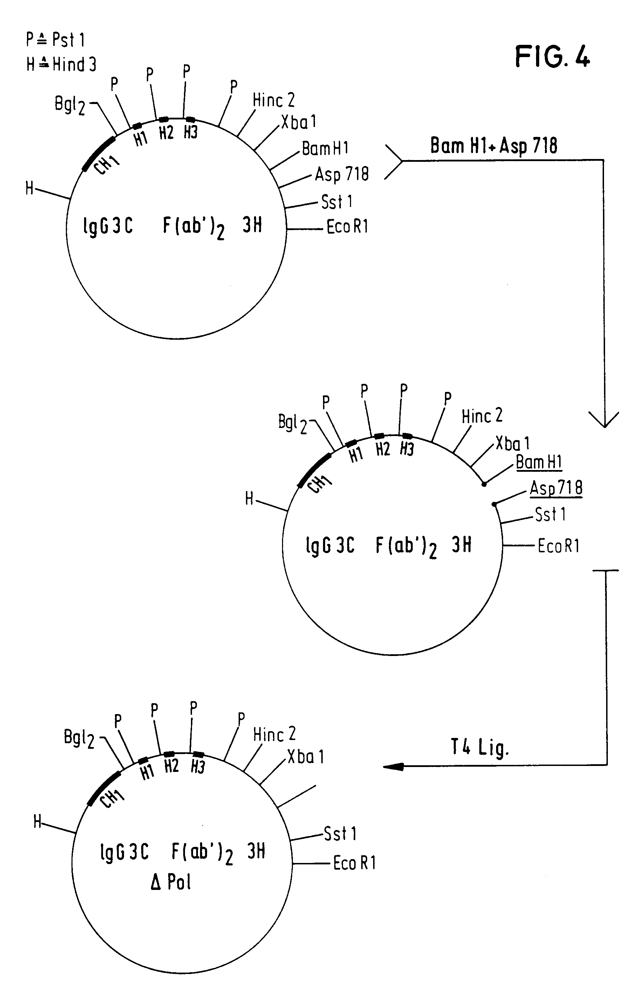

Der Plasmidklon mit dem C_{H1} und drei "Hinge-Exons" (F(ab′)₂ 3H) wurde dann mit BamH1 und Asp718 gespalten, die Schnittstellen wurden aufgefüllt und mit T₄ Ligase religiert (Fig. 4). Dadurch wird der pUC19 Polylinker zwischen der XbaI und der Sst1 Schnittstelle deletiert.

### I B) Vorbereitung des HLA B27 Gens

### Beispiel 3

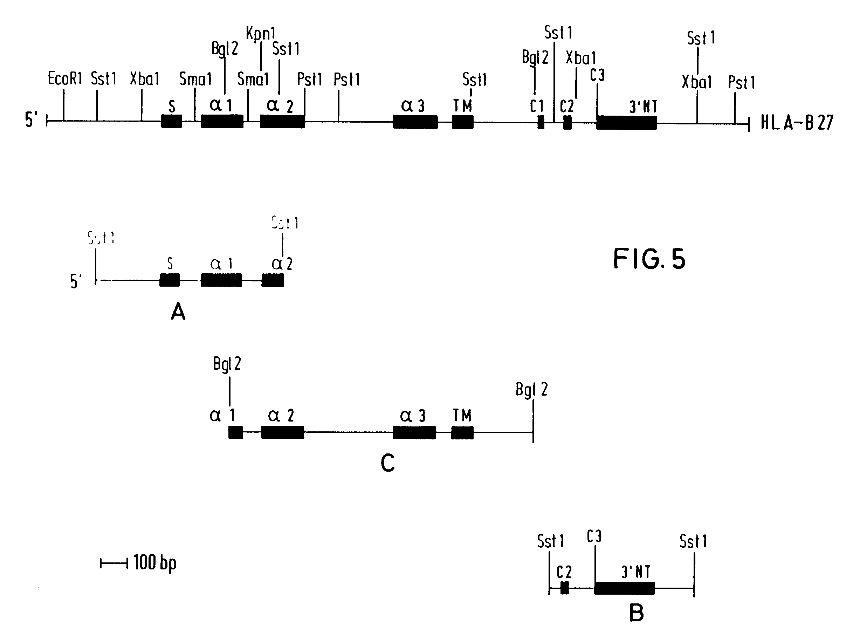

Ein HLA B27w Gen wurde aus einer in EMBL3 Bakteriophagen klonierten genomischen Genbank (Frischauf, A.-M., a.a.O.) und Seemann, G.H.A., a.a.O.) isoliert und durch Restriktionskartierung und Nukleotidsequenzanalyse charakterisiert (Maxam, A., Gilbert, W.: Sequencing and labeled DNA with base specific chemical cleavage. Meth. Enzymol. 65, 499-560 (1986) und Sanger, F., Nicklen, S., Coulson, A.R.: DNA sequencing with chain terminating inhibitors. Proc. Natl. Acad. Sci. USA 74, 5463-5467 (1977)) (Fig.5).

Das HLA B27w Gen wurde dann mit den Restriktionsenzymen Sst1 und BglII verdaut und in die Sst1- bzw. BamHI-Schnittstellen von pUC19 subkloniert. Plasmidklone mit den Subfragmenten A, B und C (Fig. 5) wurden isoliert.

### Beispiel 4

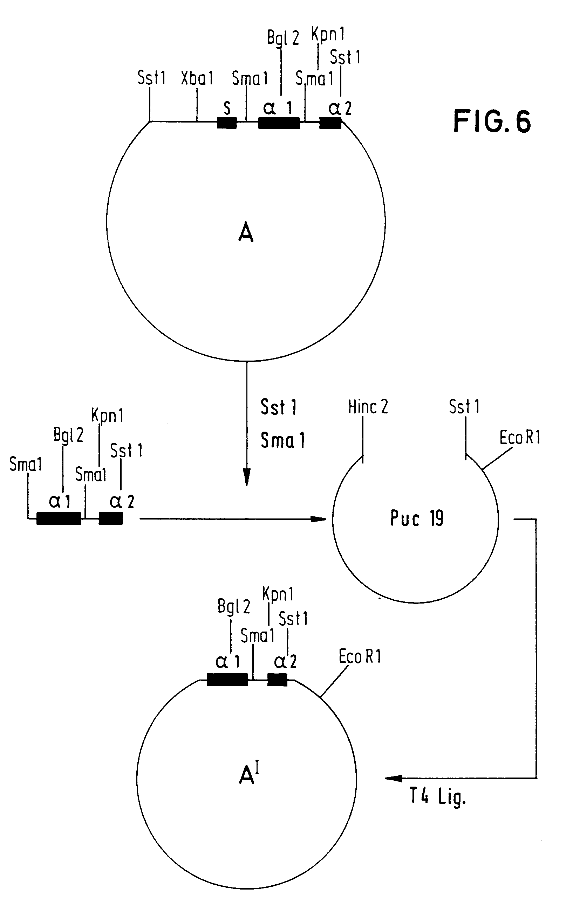

Das Plasmid mit dem Subfragment A wurde mit Sst1 vollständig und mit SmaI partiell gespalten und nach Auftrennung auf einem Agarosegel das Fragment A′ (Fig. 6) in einem mit HindII und Sst1 gespaltenen pUC19 Plasmid kloniert.

### Beispiel 5

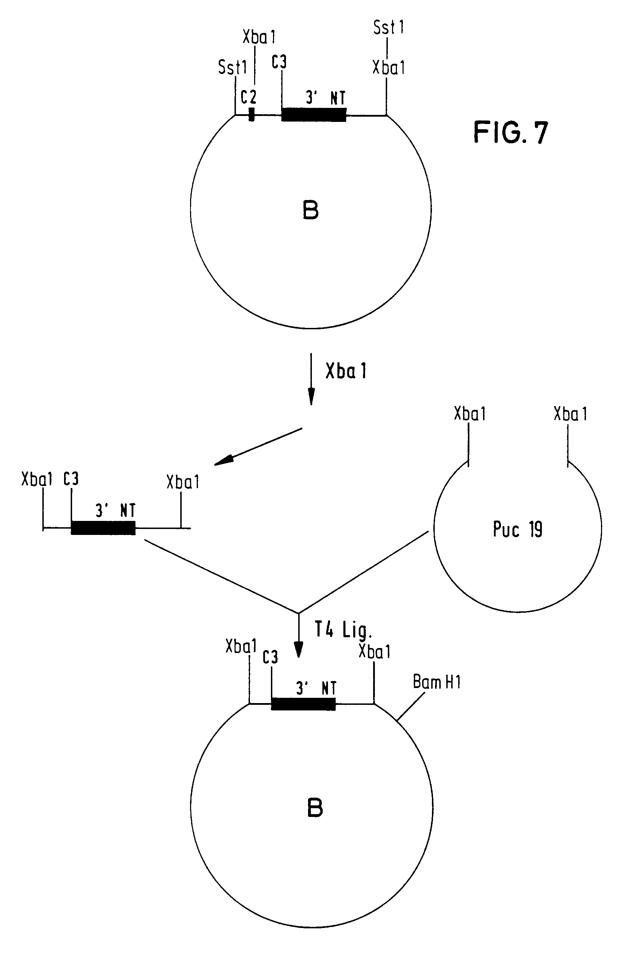

Das Plasmid mit dem Subfragment B wurde XbaI verdaut und das entstehende XbaI Insert (B′) in einen XbaI gespaltenen pUC19 Plasmid kloniert (Fig. 7).

### Beispiel 6

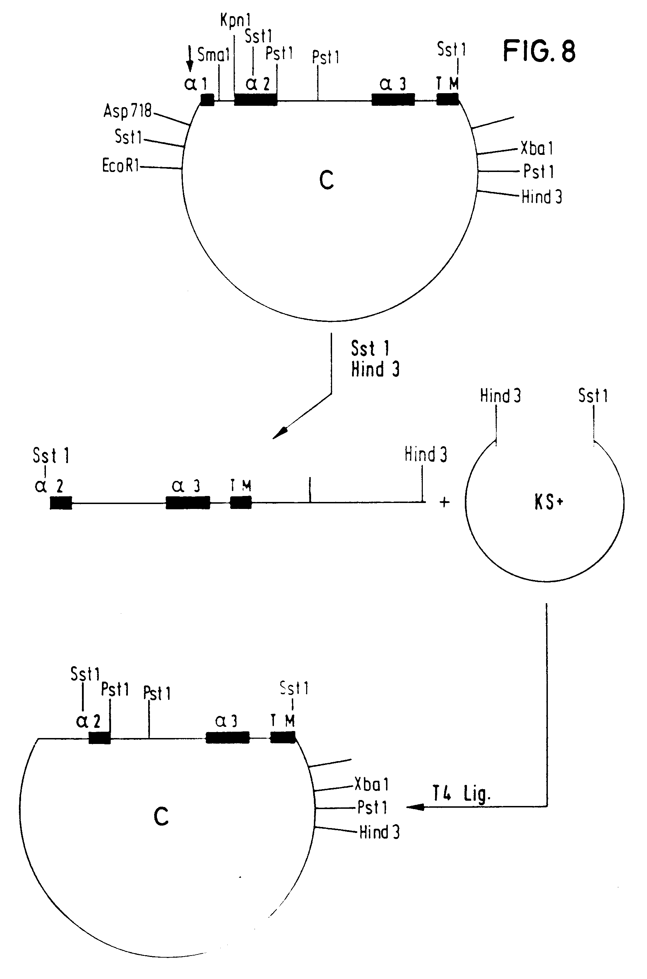

Das Plasmid mit dem Subfragment C wurde mit HindIII vollständig und mit Sst1 partiell gespalten und das Fragment, das sich im HLA B27w Gen an das Fragment A anschließt, (C′) nach Auftrennung auf einem Agarosegel isoliert und in den mit HindIII und Sst1 gespaltenen Bluescript Phasmidvektor KS+ (Stratagene, LaJolla, CA, USA) kloniert (Fig. 8).

### Beispiel 7

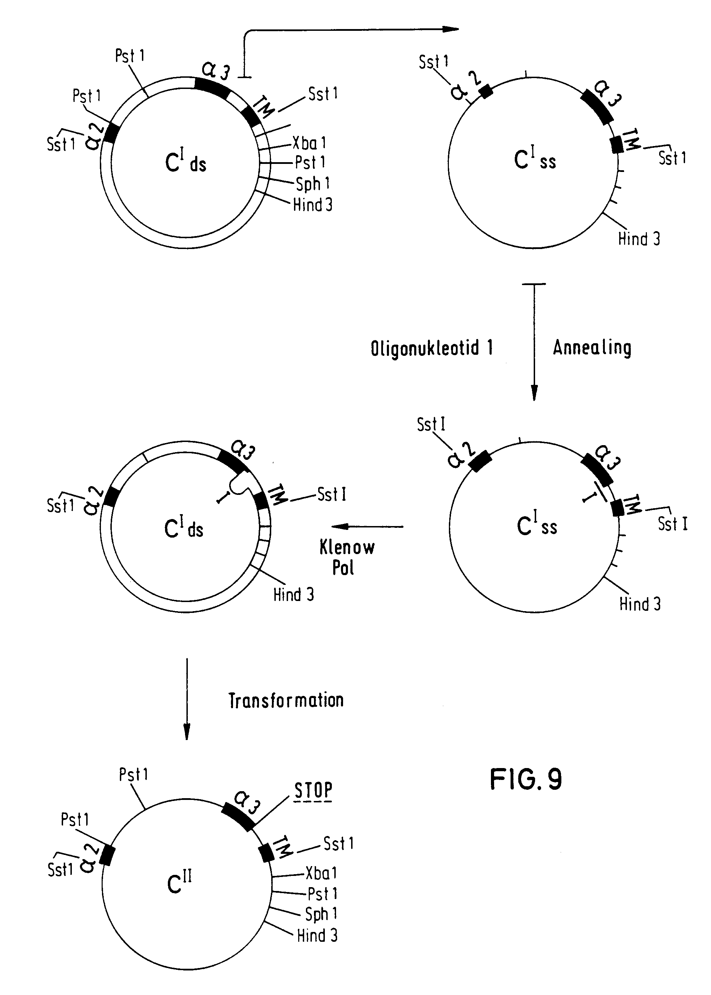

Vom KS+ Phasmidvektor C′ wurden durch Infektion mit Helferphagen VCS-M13 (Stratagene, Cat # 200251) Einzelstrangphagen präpariert und gereinigt (Stratagene: Bluescript Exo/Mung DNA sequencing system: Instruction Manual). An diese Einzelstränge wurde ein synthetisches Oligonukleotid hybridisiert (I = 5′CCTTACCTCATCTCAGG3′) und der Rest des zweiten Stranges mit Hilfe von Klenow Polymerase synthetisiert. Nach Transformation der auf diese Weise erzeugten Doppelstrang Phasmide in XL-Blue Bakterien wurden von den entstandenen Plasmidklonen wieder durch Infektion mit Helferphagen Einzelstrangphagen erzeugt und mit Hilfe eines Oligonukleotidprimers II (5′TGAGGGCTCCTGCTT3′) die Nukleotidsequenz bestimmt (Sanger, F. et al., a.a.O.). Ein Klon, bei dem das Codon TGG (Aminosäure 274) am 3′ Ende des alpha3 Exons zu einem Stop Codon (TGA) mutiert war, wurde identifiziert (C˝) (Fig. 9).

### Beispiel 8

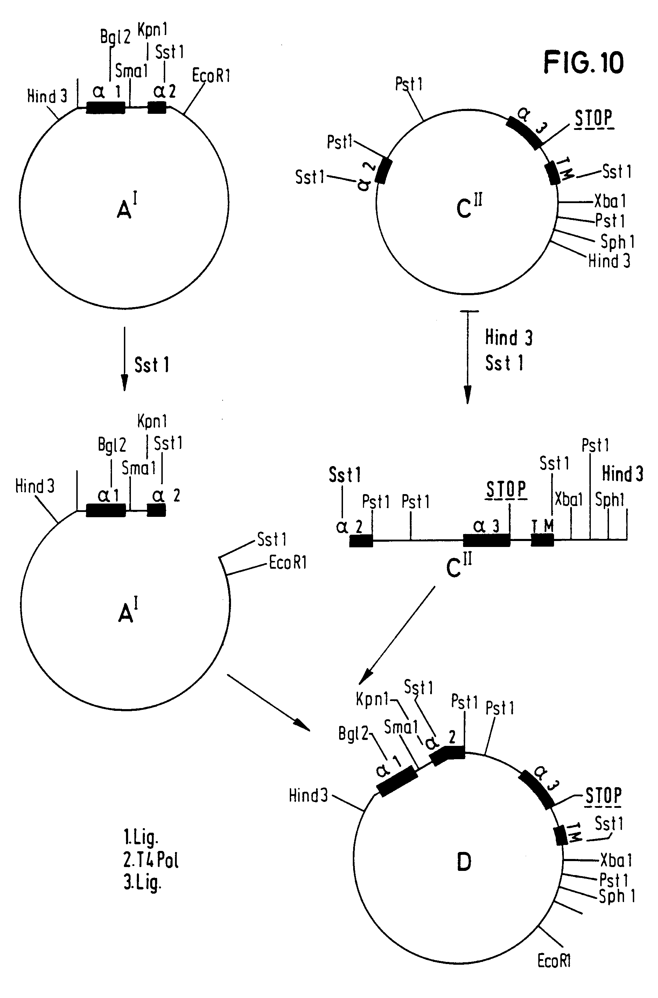

Das Plasmid mit dem Fragment A′ wurde mit Sst1 gespalten und mit dem durch eine vollständige HindIII und partielle Sst1 Spaltung des Phasmidklones C˝ erzeugten und nach Auftrennung auf einem Agarosegel isolierten C˝ Fragment ligiert. Nach 30 Min. Ligation bei 14°C wurden die nicht ligierten Enden mit T₄-Polymerase aufgefüllt und anschließend noch einmal ligiert. Durch Restriktionskartierung wurde das Plasmid D identifiziert (Fig. 10), bei dem das Fragment A′ mit dem Fragment C˝ über die Sst1-Schnittstelle im alpha2 Exon verbunden ist.

### Beispiel 9

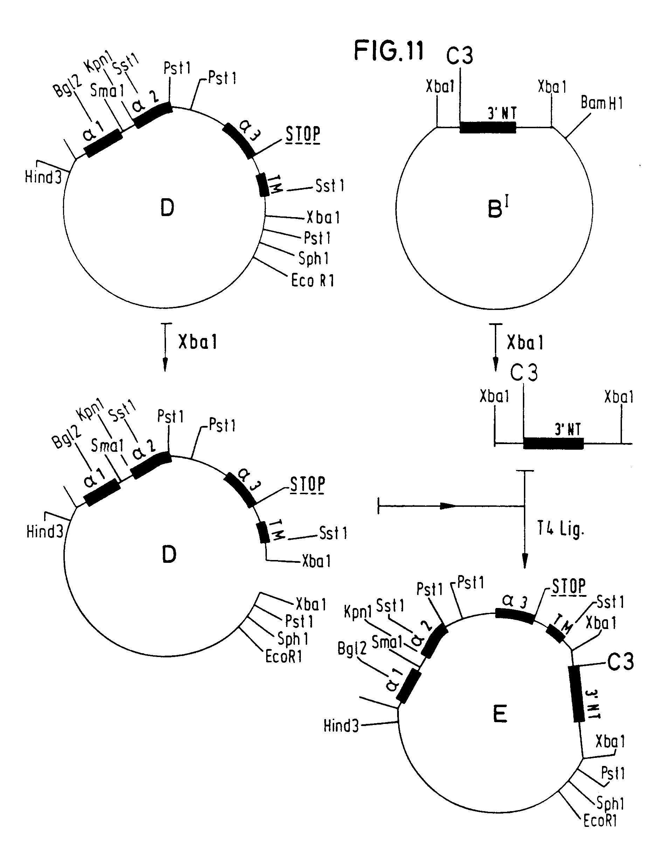

Das Plasmid mit dem Fragment D wurde mit XbaI gespalten und mit dem Fragment B′, das mit XbaI aus dem Plasmid B′ ausgeschnitten und nach Auftrennung auf einem Agarosegel gereinigt worden war, ligiert (Fig. 11). Mit Hilfe von Nukleotidsequenzanalysen (17) wurde ein Plasmid identifiziert (E), bei dem das B′ Fragment in der richtigen 5′-3′ Orientierung an das Fragment D ligiert ist.

### C) Fusion des modifizierten HLA B27w Gens mit dem IgG3 C F (ab′)₂ 3H Genfragment

### Beispiel 10

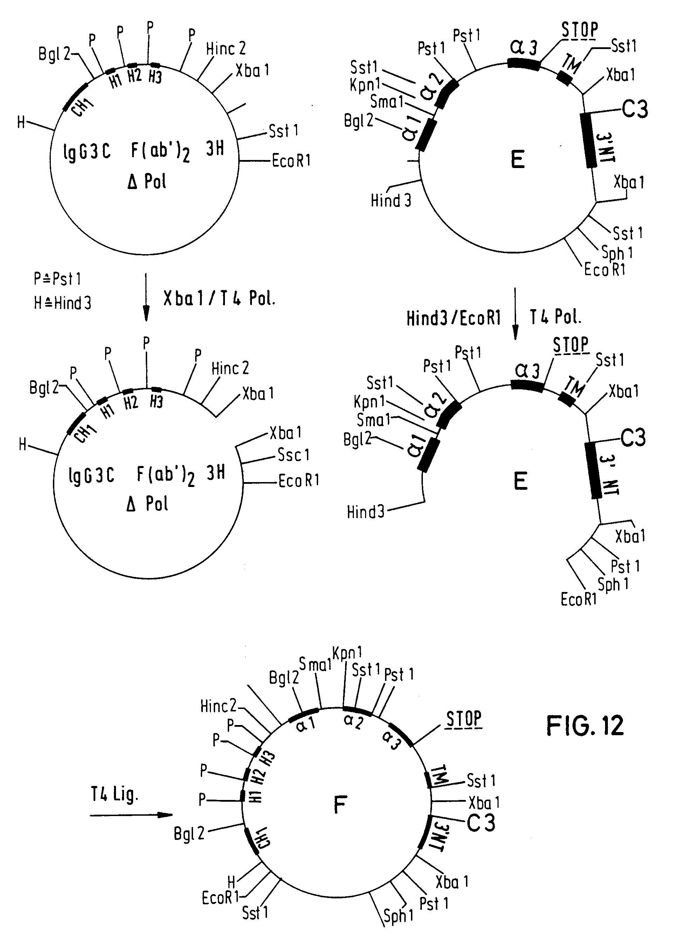

Das Fragment E wurde durch eine Spaltung mit EcoRI und HindIII aus dem Plasmid E ausgeschnitten, die Enden mit T₄-Polymerase aufgefüllt und nach Auftrennung auf einem Agarosegel gereinigt. Dieses gereinigte Fragment E wurde dann mit dem Plasmid, der das IgG3 F(ab′)₂ 3H Fragment enthält, ligiert, nachdem dieses mit XbaI gespalten und die XbaI-Enden mit T₄-Polymerase aufgefüllt worden waren (Fig. 12). Durch Restriktionskartierung wurde der Klon, der das Plasmid F enthielt, identifiziert, bei dem das modifizierte HLA B27w Gen in der richtigen 5′-3′ Orientierung mit dem F(ab′)₂ 3H Gen fusioniert ist.

### Beispiel 11

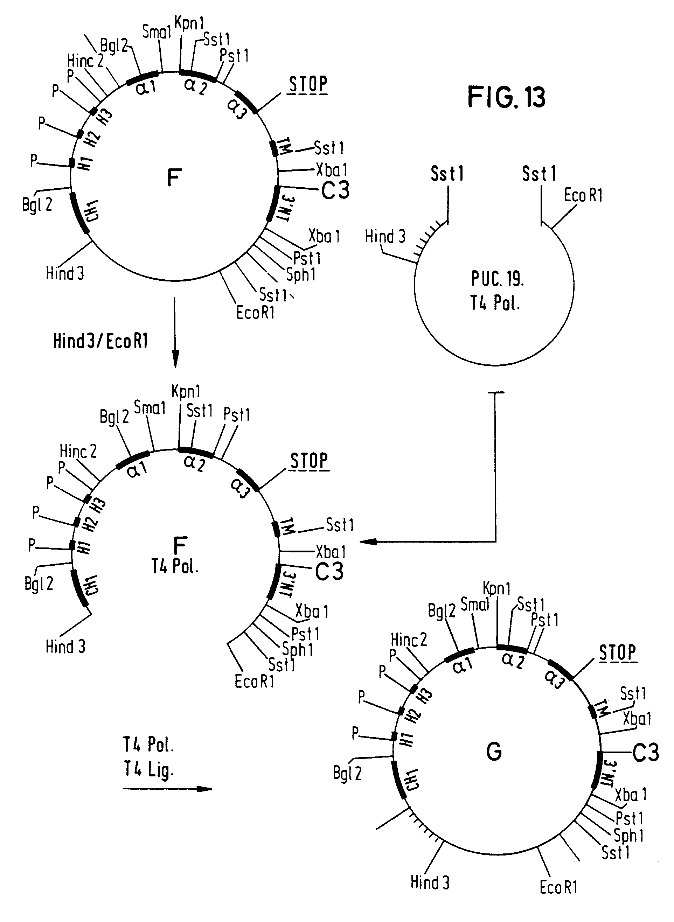

Das Fragment F wurde mit HindIII und EcoRI ausgeschnitten, um vor das 5′ Ende des Fragmentes F einen Polylinker zu setzen. Die HindIII und XbaI-Enden wurden mit T₄-Polymerase aufgefüllt und in einen pUC19 kloniert, der Sst1 gespalten und dessen Sst1 Enden mit T₄ Polymerase aufgefüllt waren. Durch Restriktionsanalysen wurde der Klon mit dem Plasmid G identifiziert, das 5′ vom Fragment F den pUC19 Polylinker besitzt. (Fig. 13).

### Beispiel 12

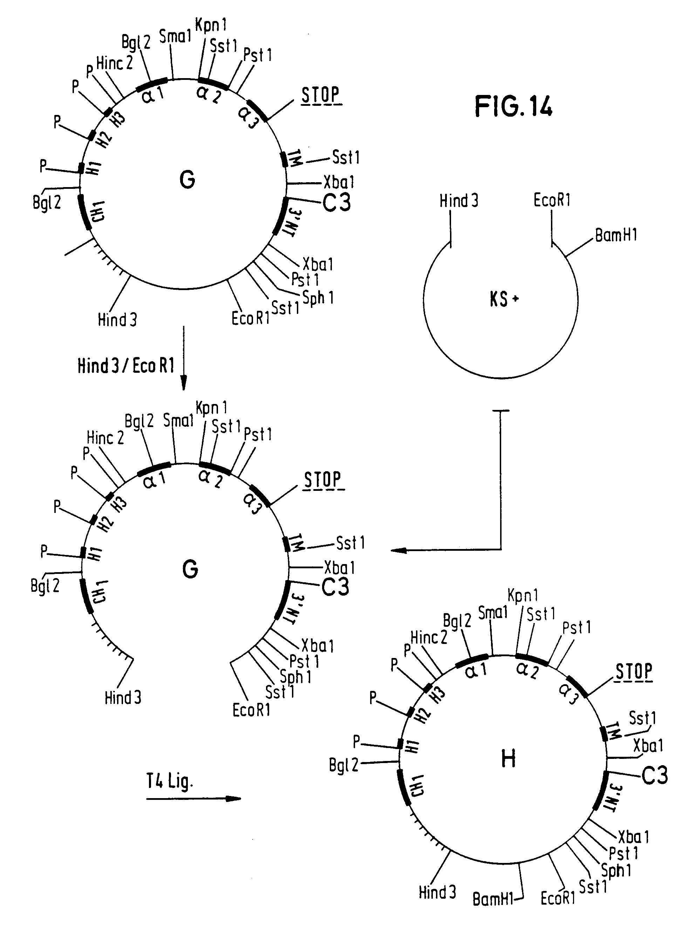

Das Plasmid G wurde mit HindIII und EcoRI gespalten, das Insert mit dem IgG F(ab′)₂ HLA B27w Fusionsgen isoliert und in einen mit HindIII und EcoRI gespaltenen Bluescript KS+ Phasmidvektor (Stratagene: Bluescript Exo/Mung DNA sequencing system: Instruction Manual) kloniert (Fig.14).

### Beispiel 13

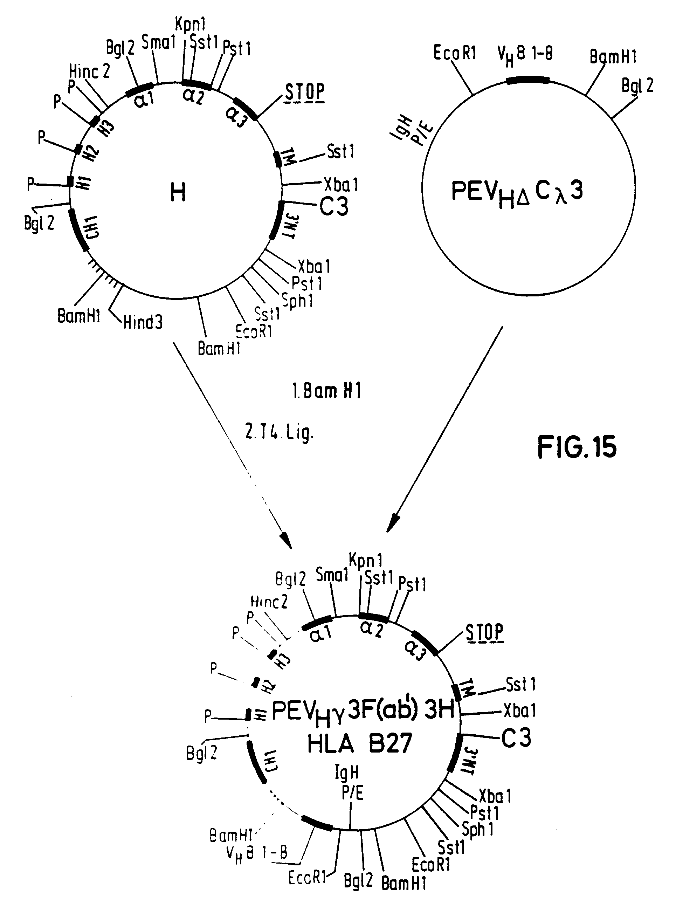

Das aus dieser Klonierung resultierende Plasmid H wurde dann mit BamHI gespalten und das Insert in den mit BamHI gespaltenen eukaryontischen Expressionsvektor pEV_{H} kloniert (Simon, T., Rajewsky, K., Nucl. Acids Res. 16, 354, (1988)), der die IgG H Promotor/Enhancer Sequenzen und das vom NP-spezifischen Maus mAK B/1-8 stammende V_{H} Gen enthält (Fig. 15) (Neuberger, M.N.: EMBO Journal 2, 1375-1378 (1983)). Durch Restriktionsanalyse wurde das Plasmid I identifiziert, bei dem das IgG 3 F(ab′)₂ HLA B27w Fusionsgen in der korrekten 5′-3′ Orientierung hinter das V_{H} Gen kloniert ist.

Das mAK/HLA B27w Fusionsgen besitzt nun intakte 5′ und 3′ Enden mit allen für die Expression in eukaryontischen Zellen erforderlichen Signalen. Das Konstrukt wird, wie eingangs gesagt, in jeder Myelomzelle ausgeprägt und sezerniert, die ein humanes β₂-Microglobulin und eine leichte Kette eines Immunglobulins enthält, deren V Gen mit V_{H} B/1-8 eine NP-Bindungsstelle bildet, wie z.B. die Maus-Myelomzelle J 558L (Oi, V.T., Morrison, S.L., Herzenberg, L.A., Berg, P., Proc. Natl. Acad. Sci. USA 80, 825 (1983)).

### II Die Beispiele 14 bis 17 zeigen die Konstruktion eines HLA B27/MAK Fusionsgen mit dem HLA B27 Anteil am 5′ Ende des monoklonalen Antikörpers

### A) Vorbereitung des HLA B27 Gens

### Beispiel 14

Ein HLA B27w Gen wurde aus einer in EMBL3 Bakteriophagen klonierten genomischen Genbank (Frischauf et al., a.a.O. und Seemann, G.H.A., a.a.O.) isoliert und durch Restriktionskartierung und Nukleotidsequenzanalyse charakterisiert (Maxam et al., a.a.O.) und (Sanger et al., a.a.O.) (Fig. 5).

Das HLA B27w Gen wurde dann mit den Restriktionsenzymen Sst1 und BglII verdaut und in die Sst1 bzw. BamH1-Schnittstellen von pUC19 subkloniert. Plasmidklone mit den Subfragmenten A, B und C (Fig. 5) wurden isoliert.

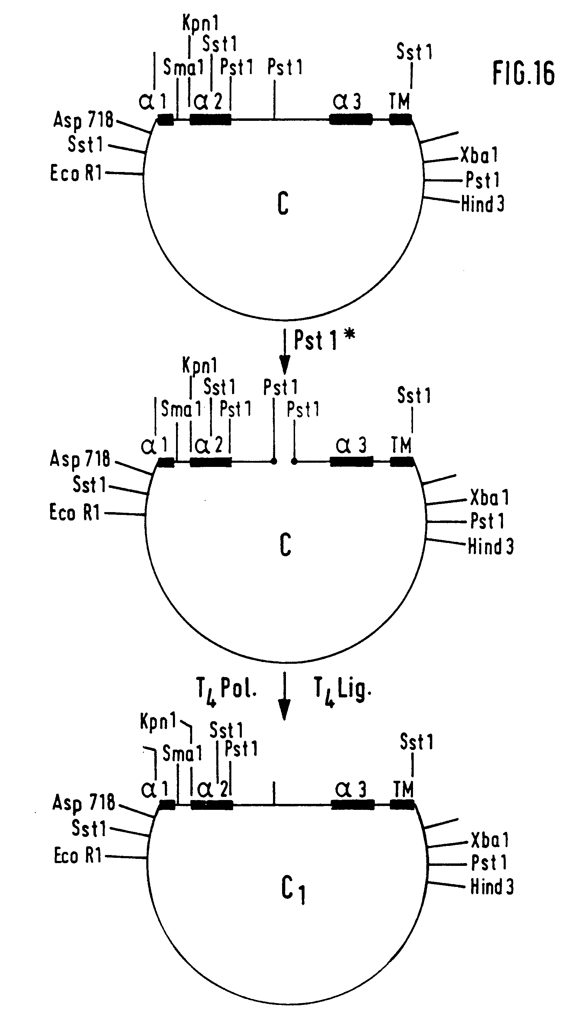

Das Plasmid mit dem HLA B27 Subklon C wurde mit Pst1 partiell gespalten. Mit T₄-Polymerase wurden unter Zugaben von dGTP die überhängenden 3′-Enden der Pst1-Schnittstellen entfernt und mit T₄-Ligase religiert. Durch Restriktionsanalyse wurde der Plasmidklon C1 identifiziert, der keine Pst1-Schnittstelle im Intron zwischen dem alpha2-und alpha3-Exon enthält (Fig. 16).

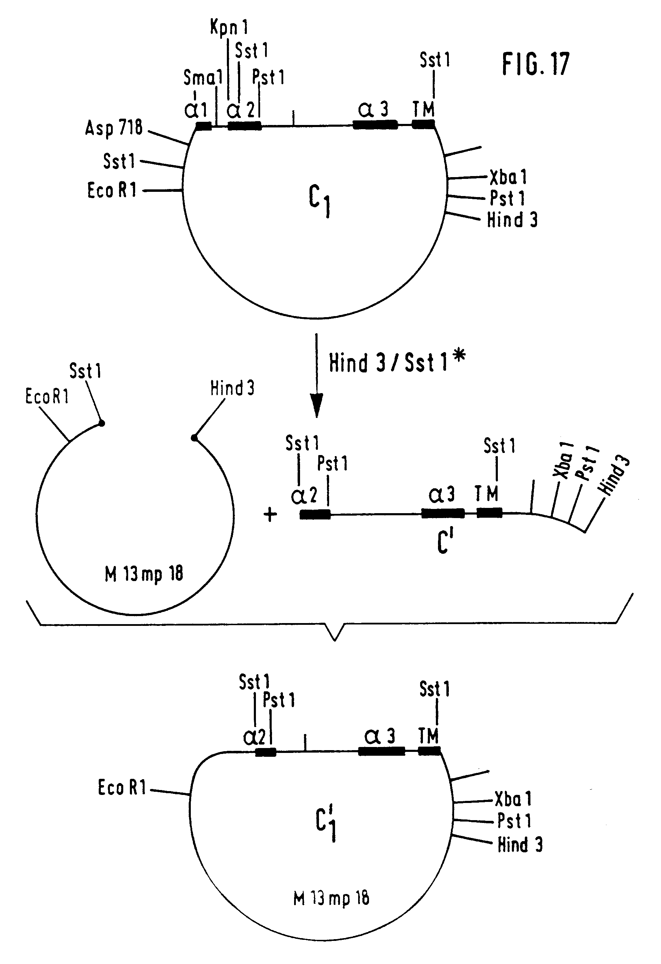

Der Plasmidklon C1 wurde mit Sst1 partiell und mit HindIII vollständig gespalten, das C1′-Fragment isoliert und in einen Sst1 und HindIII gespaltenen doppelsträngigen M13 mp18 Vektor kloniert. Der M13 Klon C1′ mit dem C1′ Fragment wurde durch Bestimmung der Nukleinsäuresequenz des Inserts identifiziert (Fig. 17).

Von dem C1′ M13 mp18 Phagen wurden in dem Bakterienstamm CJ236 Einzelstrangphagen nach dem Protokoll des Bio-Rad Muta-Gene M13 Mutagenese Kits isoliert, die Uracile enthielten. An diese Einzelstrangphagen wurde ein Oligonukleotid (Oligonukleotid III) mit der Sequenz ^{5′} GCGCGCTGGAGCGTCTC^{3′} unter der Zugabe hybridisiert und von T₄-Polymerase, dNTP und T₄-Ligase der zweite Strang synthetisiert.

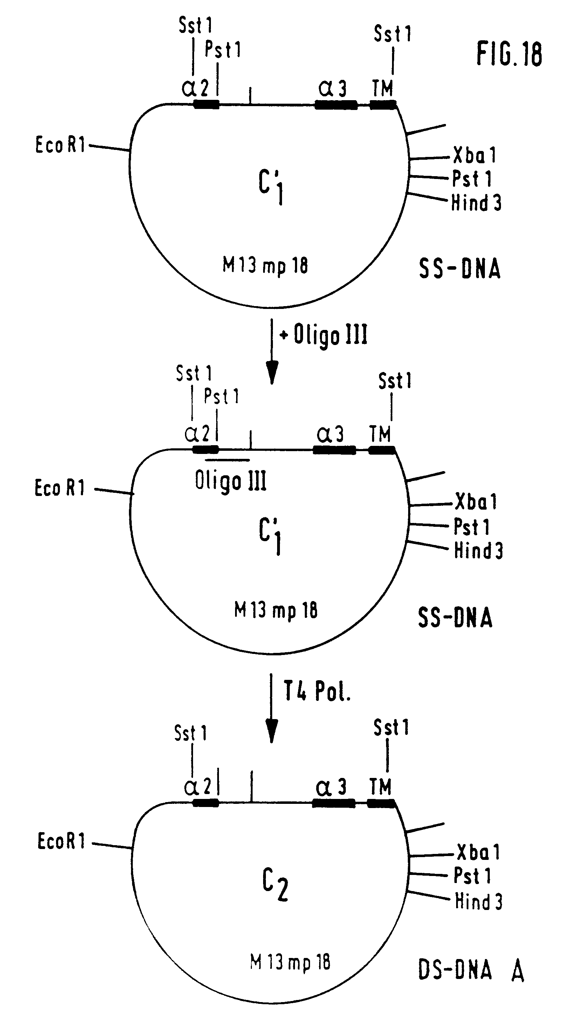

Nach Infektion des Bakterienstammes MV 1190 wurde der mutierte Klon C2 durch Restriktionsanalyse der M13 mp18 Doppelstrang DNAs identifiziert und durch Nukleinsäuresequenzanalyse bestätigt (Fig. 18). Durch die Mutagenese wurde die Pst1 Restriktionsschnittstelle im alpha2-Exon zerstört, ohne das Leseraster oder die kodierte Aminosäuresequenz zu verändern.

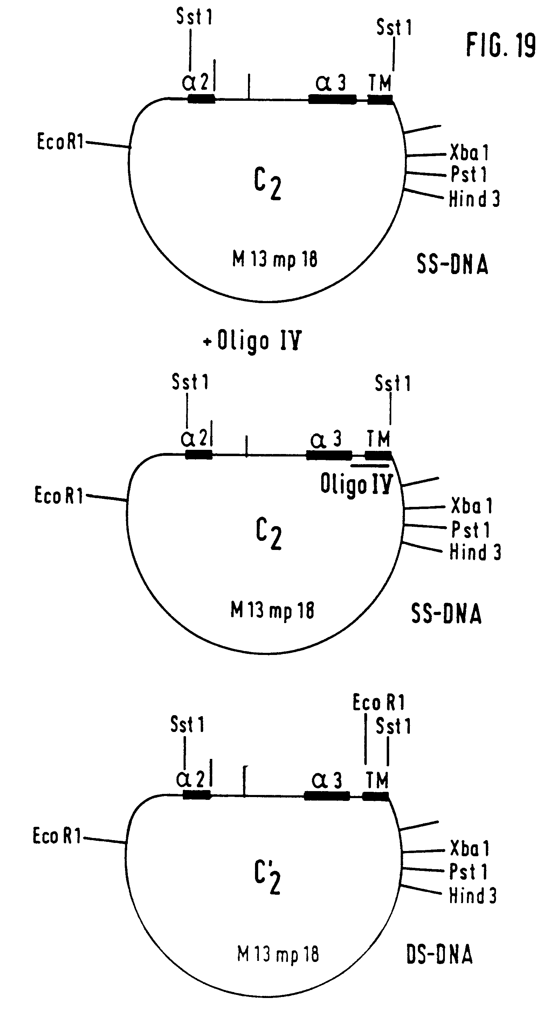

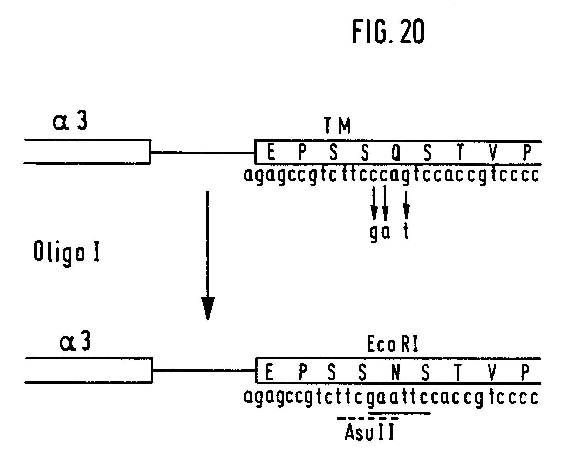

Vom M13 Klon C2 wurden im Bakterienstamm CJ236 wiederum Einzelstrangphagen produziert und mit dem Oligonukleotid IV (Oligonukleotid IV = ^{5′}GGGGACGGTGGAATTCGAAGACGGCTC^{3′}) hybridisiert. Dann wurde mit T₄-Polymerase, T₄-Ligase und dNTP der zweite Strang synthetisiert. Nach Transformation in MV 1190 Bakterien wurde der M13 mp18 Klon C2′ durch Restriktionsanalyse identifiziert und durch Nukleotidsequenzanalyse die korrekte Mutation verifiziert (Fig.19). Durch diese Mutagenese wurde in das TM Exon des HLA B27 Gens eine EcoRI und eine AsuII-Schnittstelle eingeführt und die Aminosäure 279 von Glutamin in Asparagin umgewandelt (Fig. 20).

### Beispiel 15

Der Plasmidklon mit dem Subfragment B wurde XbaI verdaut und das entstehende XbaI-Insert (B′) in einen durch XbaI gespaltenen pUC19 Plasmid kloniert (Fig. 7).

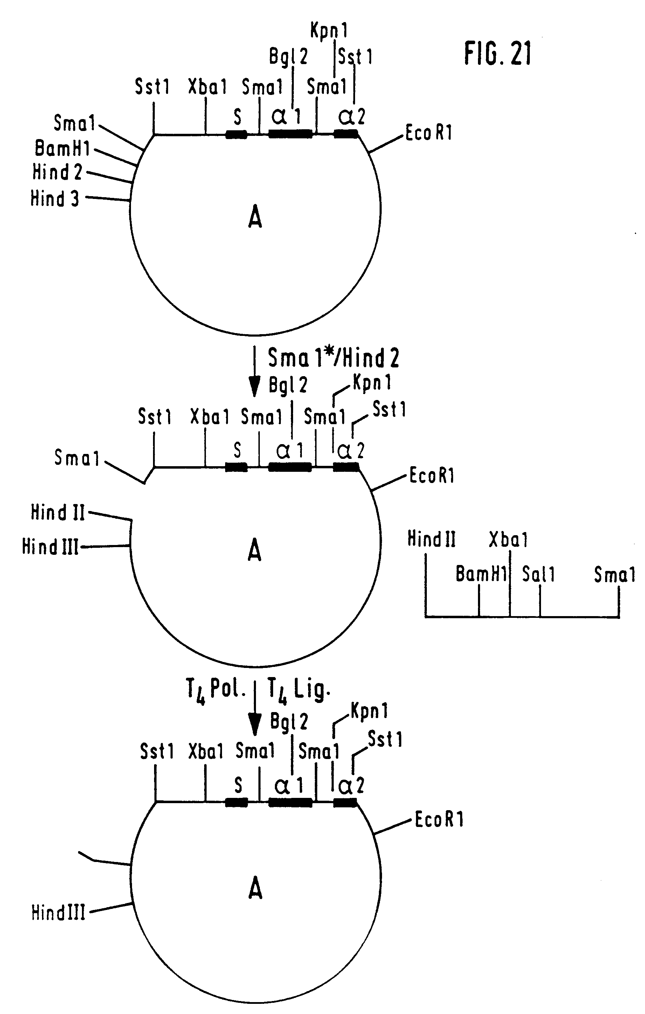

Der Plasmidklon mit dem Fragment A wurde mit HindII vollständig und SmaI partiell gespalten und religiert. Durch Restriktionsanalyse wurde der Klon A′ identifiziert, bei dem ein Teil des pUC19 Polylinkers deletiert ist (Fig. 21).

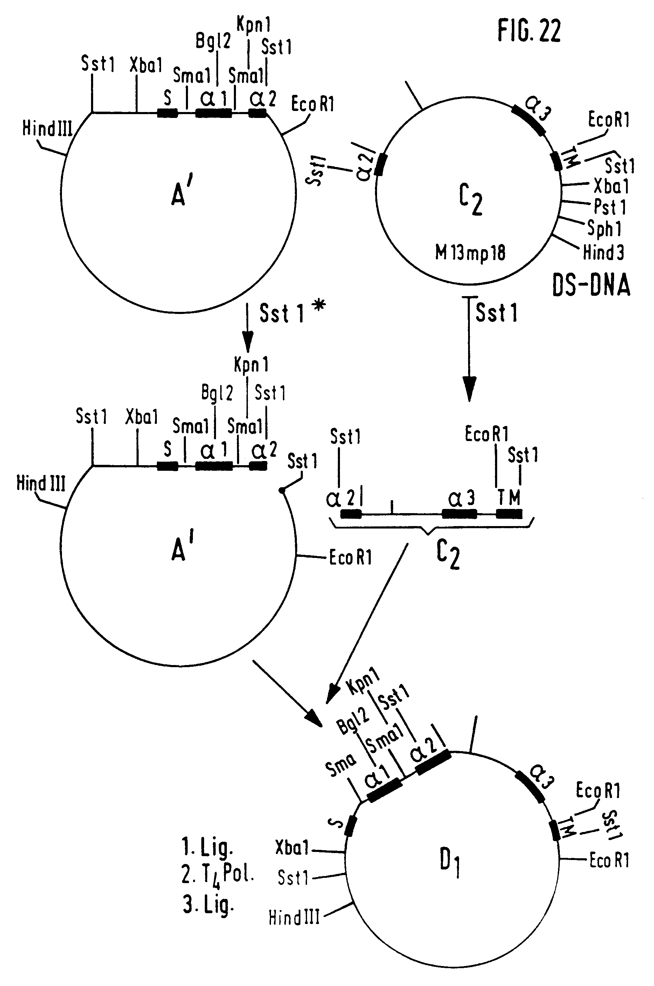

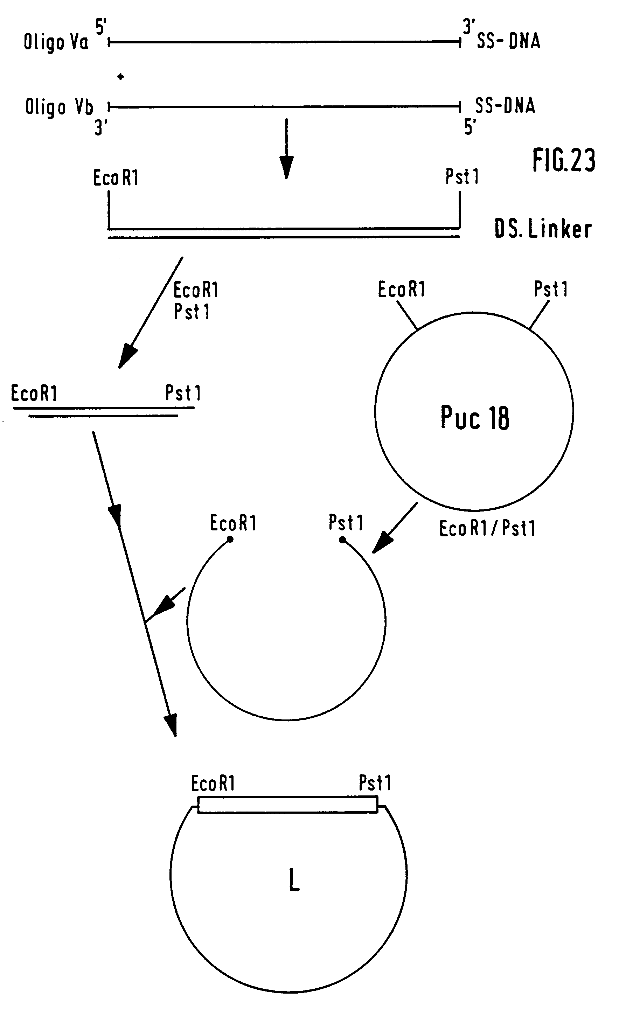

Der Plasmidklon A′ wurde partiell mit Sst1 gespalten und mit dem durch eine Sst1-Spaltung des Plasmidklones C2 erzeugten und nach Auftrennung auf einem Agarosegel isolierten C2-Fragment ligiert. Durch Restriktionskartierung wurde das Plasmid D₁ identifiziert (Fig. 22), bei dem das Fragment A mit dem Fragment C2 über die Sst1-Schnittstelle im alpha2-Exon verbunden ist. Das 5′-Ende des HLA B27w Gens ist damit vollständig.
Konstruktion des Linkers:
Es wurden zwei Oligonukleotide synthetisiert:
Oligonukleotid Va: 
5' TCGAATTCCG GCGAGGCAGC TCCCGCAGCT GCACCCGCAG CAGCCGCAGC
AGGCGGGCAG GTCCAACTGC AGGA 3'
Oligonukleotid Vb: 
5' TCCTGCAGTT GGACCTGCCC GCCTGCTGCG GCTGCTGCGG GTGCAGCTGC
GGGAGCTGCC TCGCCGGAAT TCGA 3'
Die beiden Oligonukleotide wurden miteinander hybridisiert. Dabei entstanden doppelsträngige DNA Fragmente, mit einer EcoRI Restriktionsschnittstelle am einen und einer Pst1 Restriktionschnittstelle am anderen Ende. Diese Fragmente wurden mit EcoRI und Pst1 gespalten und in einen EcoRI und Pst1 gespaltenen pUC19 Plasmidvektor kloniert (Fig. 23). Der Plasmidklon L wurde durch Restriktionsanalyse identifiziert und durch Nukleotidsequenzanalyse verifiziert.

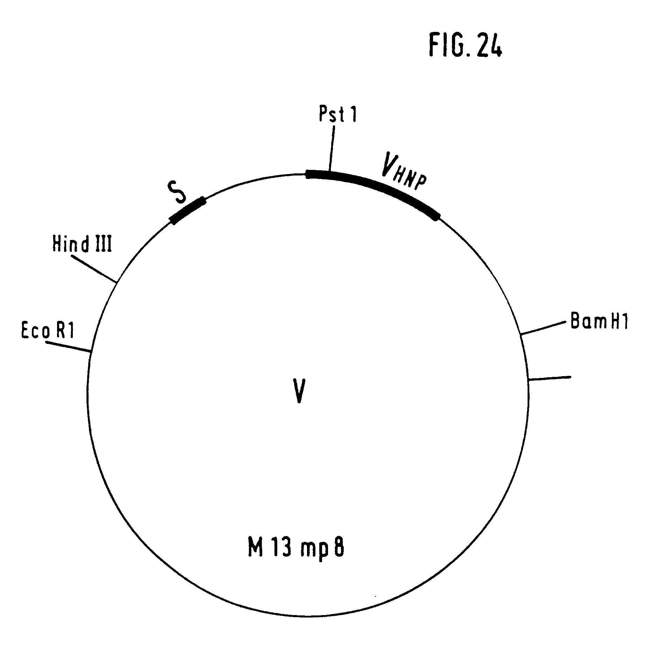

Das Immunglobulin V-Gen wurde von P.T. Jones et al. (Jones, P.T., Dear, P.H., Foote, J., Neuberger, M.S. Winter, G., Nature 321: 522, (1986)) mit Hilfe von Oligonukleotiden synthetisiert. Es enthält eine Pst1-Restriktionsschnittstelle im 5′ Bereich des Klons und ist als HindIII/BamHI Fragment in einen M13 mp8 Vektor kloniert, dessen Pst1-Schnittstelle durch Spaltung, Entfernung der überhängenden Enden und Religation zerstört worden war (Fig. 24).

### II B) Vorbereitung des mAK C-Gen Anteils:

### Beispiel 16

Ein humanes IgG3 C-Gen wurde aus einer humanen Genbank in EMBL3 Phagen isoliert (Frischauf et al., a.a.O. und Seemann et al., a.a.O.) und als 3,1 kb großes HindIII/SphI Fragment in den Plasmidvektor pUC19 subkloniert (Klon 54.1.24) (Fig. 2).

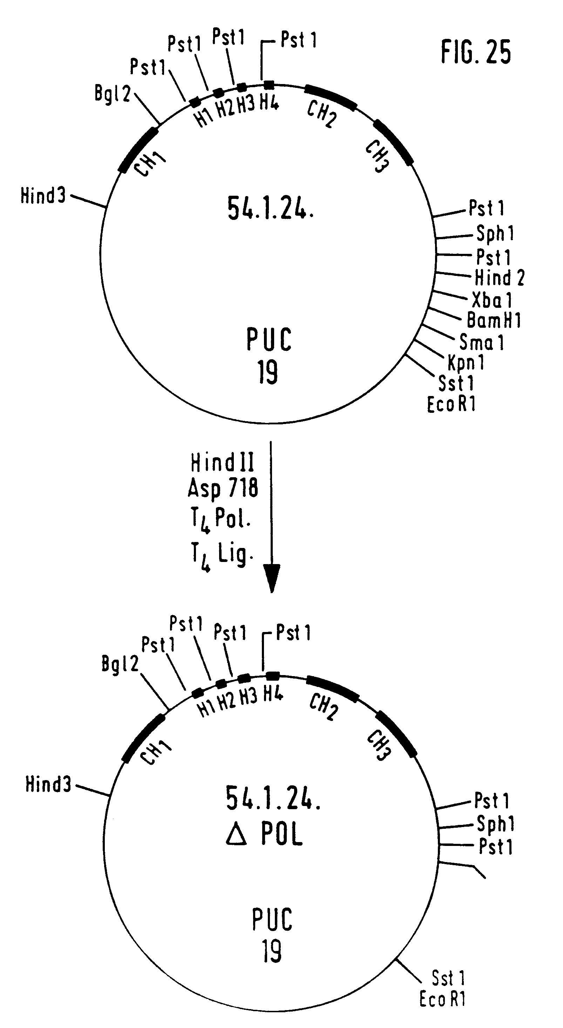

Der Plasmidklon 54.1.24 wurde mit HindII und Asp718 gespalten, die überhängenden Enden der Asp718 Schnittstelle mit T₄-Polymerase entfernt und mit T₄-Ligase religiert. Durch Restriktionsanalyse und Nukleinsäuresequenzbestimmung wurde der Klon 54.1.24 Delta Pol identifiziert, der außer SphI, PstI, Sst1 und EcoRI keine Restriktionsschnittstellen 3′ des humanen IgG3 C-Gens mehr enthält (Fig. 25).

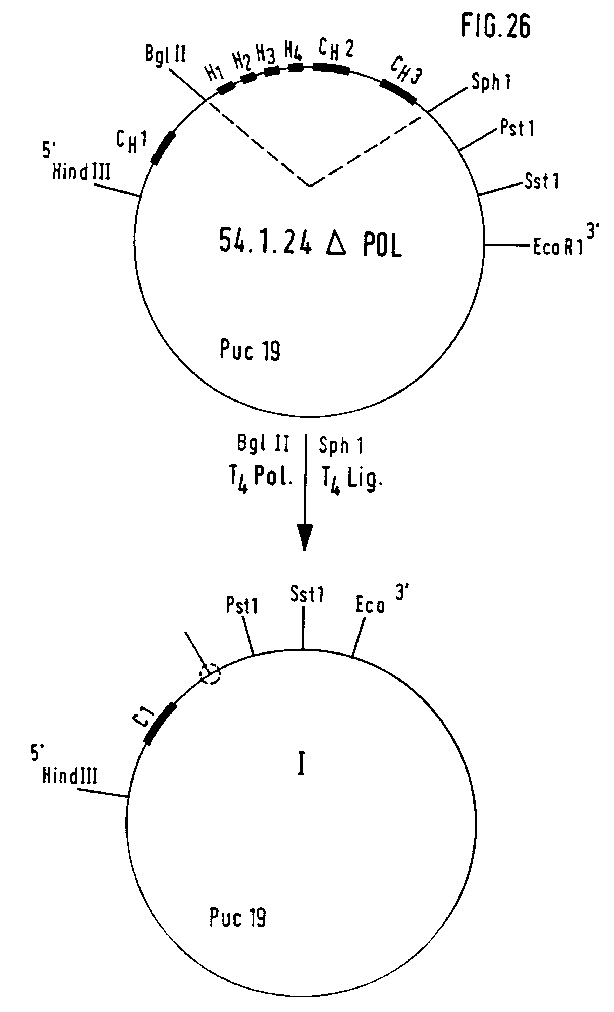

Der Plasmidklon 54.1.24 Delta Pol wurde mit Bg12 und SphI verdaut. Die überhängenden Enden wurden mit T₄-Polymerase entfernt und mit T₄-Ligase religiert. Durch Restriktionsanalyse und Nukleinsäuresequenzbestimmung wurde der Klon I identifiziert, der nur noch das CH₁-Exon des humanen IgG3 C-Gens enthält (Fig. 26).

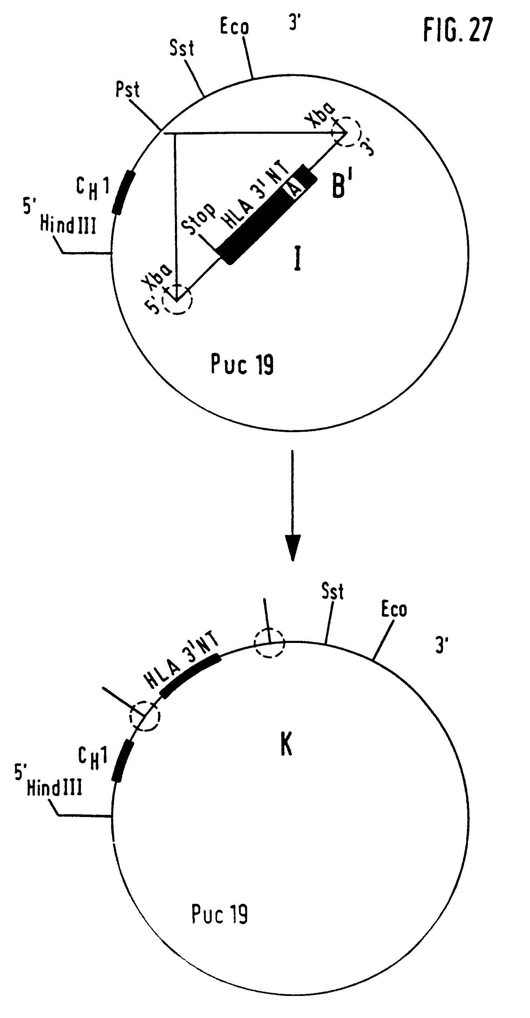

Der Plasmidklon I wurde mit Pst1 gespalten und die überhängenden Enden mit T₄-Polymerase entfernt. In die so entstandenen glatten Enden wurde das mit XbaI ausgeschnittene und mit T₄-Polymerase zu glatten Enden aufgefüllte B′ Insert ligiert. Durch Restriktionsanalyse und Nukleinsäuresequenzbestimmung wurde der Klon K identifiziert (Fig. 27), der ein humanes IgG₃C_{H}1 Exon und ein 3′ Ende eines HLA Klasse I Gens enthält.

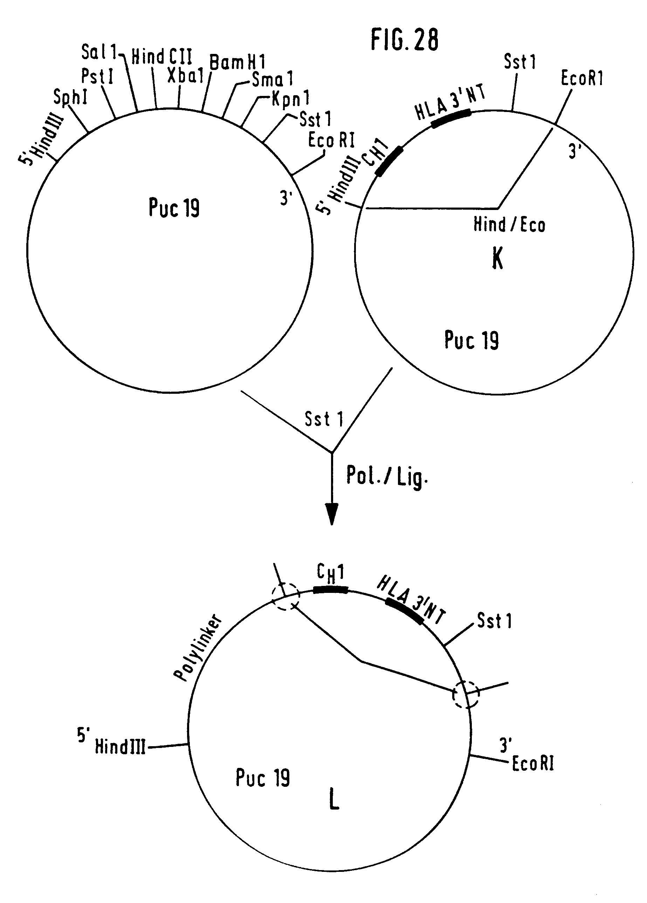

Der Plasmidklon K wurde mit HindIII und EcoRI gespalten, die überhängenden Enden entfernt und das Insert in einen mit Sst1 gespaltenen pUC19 Plasmid, dessen Enden ebenfalls glatt gemacht worden waren, ligiert. Der Klon L wurde identifiziert, der den Polylinker des pUC19 Vektors 5′ vom C_{H}1 Exon trägt (Fig. 28).

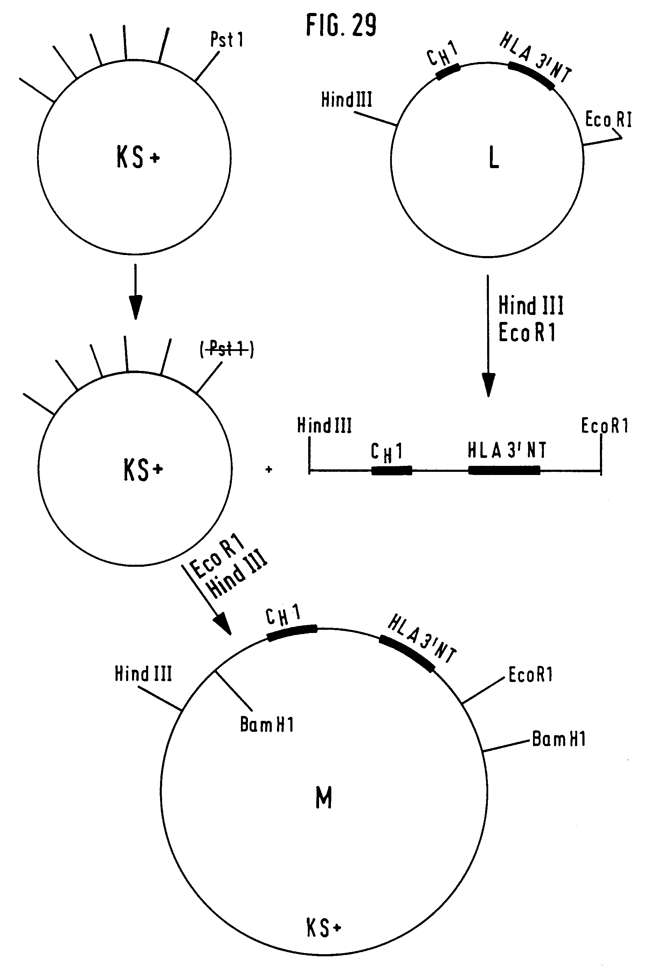

Der Plasmidklon L wurde mit EcoRI und HindIII gespalten, das Insert gereinigt und in einen HindIII und EcoRI gespaltenen KS⁺ Vektor (Stratagene; Bluescript Exo/Mung DNA Sequencing System) ligiert, dessen PstI-Schnittstelle zuvor durch Spaltung mit PstI, T₄-Polymerasebehandlung und Religation zerstört worden war. Der Klon M wurde identifiziert, aus dem man das humane C_{H}1 Exon mit dem HLA Klasse I 3′ Ende durch eine BamHI-Spaltung ausschneiden kann (Fig. 29).

### Beispiel 17

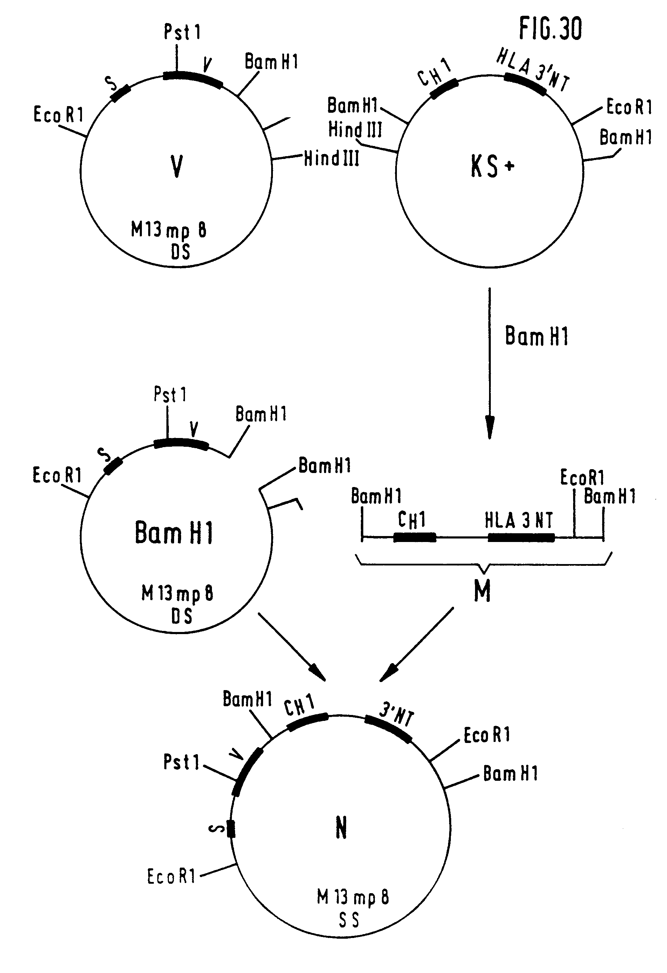

Vom M13 mp8 Klon V wurde doppelsträngige DNA hergestellt und mit BamHI gespalten. Der KS⁺ Klon M wurde mit BamHI gespalten und das Insert M gereinigt. Das M-Fragment wurde in den BamHI-gespaltenen Klon V ligiert und mit Hilfe von Nukleinsäuresequenzbestimmung der M13 Klon N identifiziert, der ein intaktes IgG3-Gen enthält (Fig. 30).

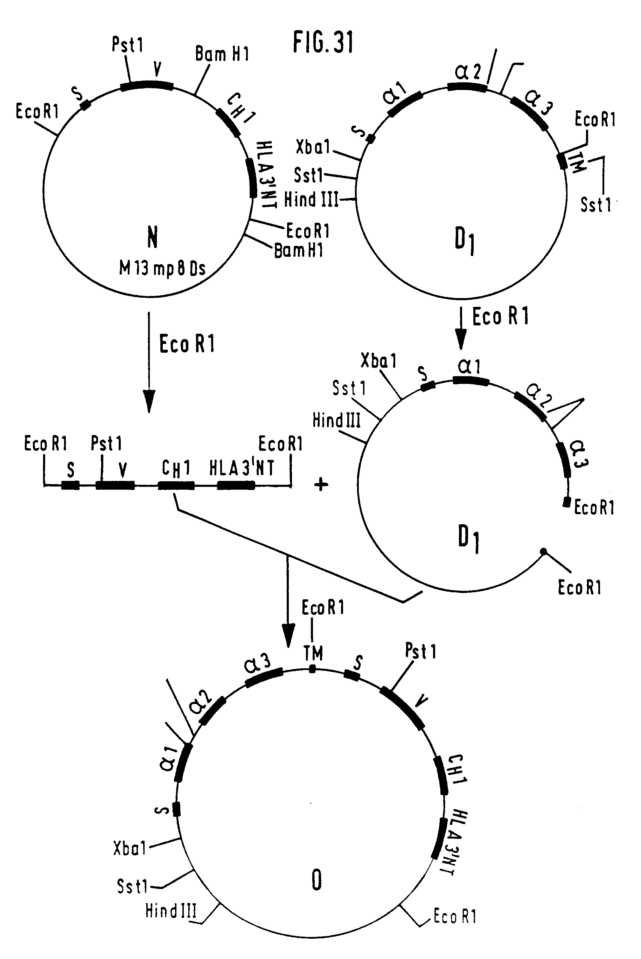

Vom M13 Klon N wurde Doppelstrang DNA hergestellt, mit EcoRI gespalten und das Insert gereinigt. Der Plasmidklon D₁ wurde mit EcoRI gespalten und mit dem Fragment N ligiert. Der Phagenklon O wurde isoliert, bei dem das Fragment N in der richtigen Orientierung in den Klon D₁ kloniert ist (Fig. 31).

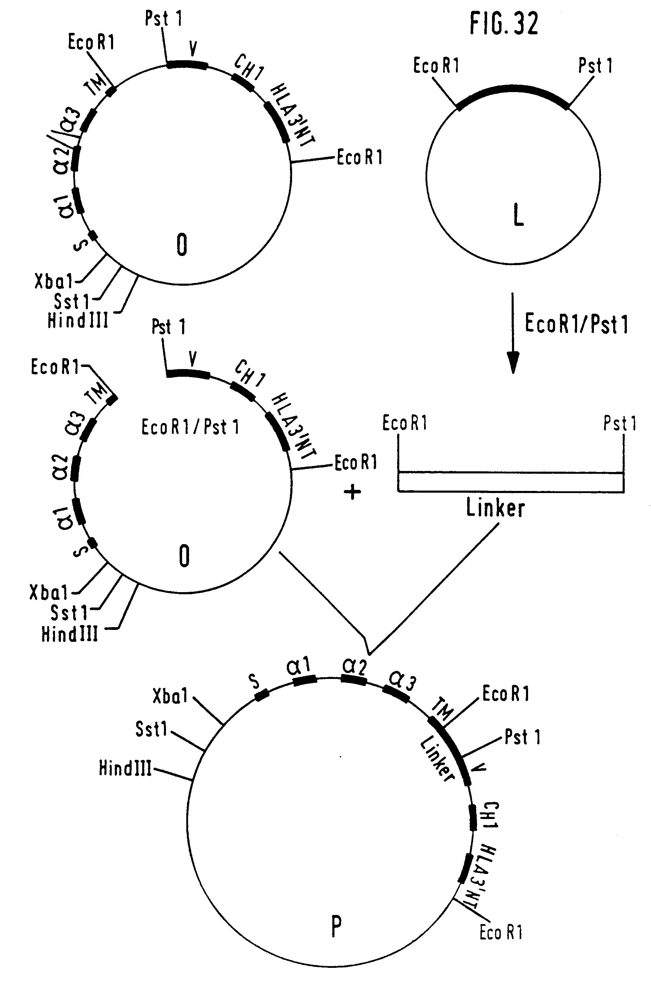

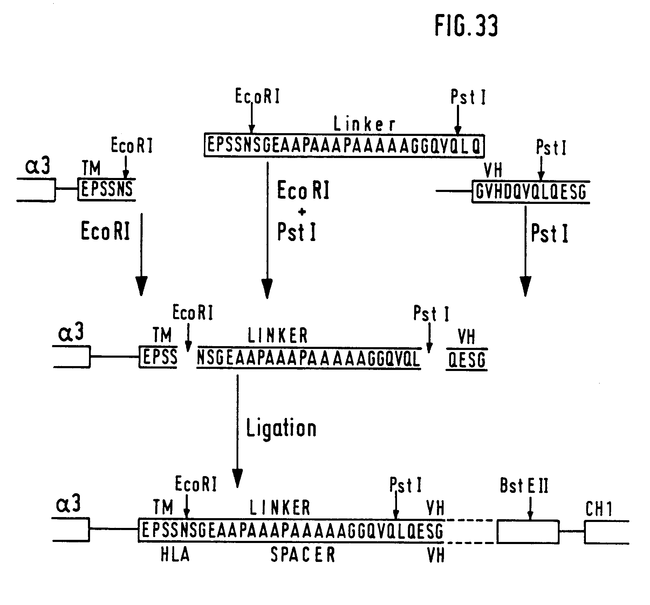

Der Plasmidklon O wurde einer vollständigen PstI-Spaltung und einer partiellen EcoRI-Spaltung unterworfen und mit dem mit EcoRI und Pst1 aus dem Plasmidvektor L ausgeschnittenen Linkerfragment ligiert. Der Plasmidklon P enthält das vollständige HLA B27w mAK Fusionsgen (Fig. 32, Fig. 33). Dieses Fusionsgen kann in humanen Zellen allein oder in Mauszellen zusammen mit dem humanen beta₂ Microglobulin-Gen ausgeprägt und sezerniert werden, wenn auch eine Immunglobulin leichte Kette in den Expressionszellen vorhanden ist.

### Legende zu Fig. 1

alpha 1, alpha 2 und alpha 3 bedeuten die Domänen der Klasse I MHC Antigenkette. Die Pfeile zeigen auf die alpha-Helices, die die Allodeterminanten tragen. CM soll die Zellmembran darstellen, C die Zelle.

### Legende zu Fig. 2 ff

EcoRI etc. steht für die Spaltung mit der jeweiligen Restriktionsendonuklease bzw. für die entsprechende Schnittstelle. BamHI / Asp718 bedeutet eine durch Religation nach Auffüllen zerstörte Restriktionsschnittstelle.

TM bedeutet Transmembranregion.

3′NT bedeutet 3′-nichttranslatiert
IgH p/E bedeutet Immunglobulin Schwere Kette-Promoter/Enhancer
^{*} bedeutet: unvollständige Verdauung
DS-DNA bedeutet: Doppelstrang DNA
SS-DNA bedeutet: Einzelstrang DNA

### Legende zu Fig. 34:

s.CTL bedeutet: syngener zytotoxischer T-Lymphozyt
TcR bedeutet: T-Zell Rezeptor
a.MHC class I bedeutet: allogenes MHC Klasse I Antigen
t.a.a. bedeutet: Tumor-assoziiertes Antigen
s.t.c. bedeutet: syngene Tumorzelle

## Patentansprüche (Patentansprüche für folgende(n) Vertragsstaat(en): AT, BE, CH, DE, FR, GB, IT, LI, LU, NL, SE)

1. Antigenkonstrukte, dadurch gekennzeichnet, daß "Major Histocompatibility Complex" (MHC) Klasse I Antigene am C- oder N-terminalen Ende mit spezifischen Trägermolekülen kovalent verbunden sind.

2. Antigenkonstrukte nach Anspruch 1, dadurch gekennzeichnet, daß "Major Histocompatibility Complex" (MHC) Klasse I Antigene am aminoterminalen Ende mit spezifischen Trägermolekülen kovalent verbunden sind.

3. Antigenkonstrukte nach Anspruch 1, dadurch gekennzeichnet, daß "Major Histocompatibility Complex" (MHC) Klasse I Antigene am C-terminalen Ende mit spezifischen Trägermolekülen kovalent verbunden sind.

4. Antigenkonstrukte nach Anspruch 1, 2 oder 3, dadurch gekennzeichnet, daß jeweils ein MHC Klasse I Antigen mit einem spezifischen Trägermolekül kovalent verbunden sind.

5. Antigenkonstrukte nach Anspruch 1, 2, 3 oder 4, dadurch gekennzeichnet, daß die spezifischen Trägermoleküle CD4 Domänen sind.

6. Antigenkonstrukte nach Anspruch 1, 2, 3 oder 4, dadurch gekennzeichnet, daß die spezifischen Trägermoleküle monoklonale Antikörper sind.

7. Antigenkonstrukte nach Anspruch 6, dadurch gekennzeichnet, daß die monoklonalen Antikörper im konstanten Teil der schweren Kette verkürzt sind.

8. Antigenkonstrukte nach Anspruch 1, 2, 3, 4, 5, 6 oder 7, dadurch gekennzeichnet, daß das MHC Klasse I Antigen HLA B27w oder HLA B27k ist.

9. Antigenkonstrukte nach Anspruch 1, 2, 3, 4, 5, 6, 7 oder 8, dadurch gekennzeichnet, daß sie gentechnisch durch Fusion der für sie kodierenden DNA's hergestellt werden.

10. Verfahren zur Herstellung von Antigenkonstrukten nach Anspruch 1, 2, 3, 4, 5, 6, 7 oder 8 dadurch gekennzeichnet, daß die erforderlichen Genteile in Form ihrer DNA fusioniert, mit geeigneten Regulationssequenzen versehen und in geeigneten Expressionssystemen exprimiert werden.

11. Verwendung der Antigenkonstrukte nach einem der Ansprüche 1 bis 11 zur Herstellung eines Arzneimittels zur Allogenisierung von Zielzellen.

12. Arzneimittel, die Antigenkonstrukte nach einem der Ansprüche 1 bis 9 enthalten.

## Patentansprüche (Patentansprüche für folgende(n) Vertragsstaat(en): ES, GR)

1. Verfahren zur Herstellung von Antigenkonstrukten aus "Major Histocompatibility Complex" (MHC) Klasse I Antigenen und spezifischen Trägermolekülen, dadurch gekennzeichnet, daß die erforderlichen Genteile in Form ihrer DNA fusioniert, mit geeigneten Regulationssequenzen versehen und in geeigneten Expressionssystemen exprimiert werden.

2. Verfahren nach Anspruch 1, dadurch gekennzeichnet, daß die erforderlichen Genteile an das 3' Ende des MHC Klasse I Gens ligiert werden.

3. Verfahren nach Anspruch 1, dadurch gekennzeichnet, daß die erforderlichen Genteile an das 5' Ende des MHC Klasse I Gens ligiert werden.

4. Verfahren nach Anspruch 1, dadurch gekennzeichnet, daß jeweils ein MHC Klasse I Antigen mit einem spezifischen Trägermolekül verknüpft ist.

5. Verfahren nach Anspruch 1, 2, 3 oder 4, dadurch gekennzeichnet, daß als spezifisches Trägermolekül CD4-Domänen eingesetzt werden.

6. Verfahren nach Anspruch 1, 2, 3 oder 4, dadurch gekennzeichnet, daß als spezifisches Trägermolekül monoklonale Antikörper eingesetzt werden.

7. Verfahren nach Anspruch 1, 2, 3, 4, 5 oder 6, dadurch gekennzeichnet, daß das HLA B27w oder HLA B27k Gen oder Molekül eingesetzt wird.

8. Verfahren nach Anspruch 6, dadurch gekennzeichnet, daß die eingesetzten monoklonalen Antikörper im konstanten Teil der schweren Kette verkürzt sind.

9. Verfahren zur Herstellung von Arzneimitteln, dadurch gekennzeichnet, daß nach Anspruch 1, 2, 3, 4, 5, 6, 7 oder 8 hergestellte Antigenkonstrukte mit üblichen Tragermaterialien gemischt werden.

## Claims (Claims for the following Contracting State(s): AT, BE, CH, DE, FR, GB, IT, LI, LU, NL, SE)

1. Antigenic constructs in which major histocompatibility complex (MHC) class I antigens are covalently bonded at the C- or N-terminal end to specific carrier molecules.

2. Antigenic constructs as claimed in claim 1, in which major histocompatibility complex (MHC) class I antigens are covalently bonded at the amino-terminal end to specific carrier molecules.

3. Antigenic constructs as claimed in claim in which major histocompatibility complex (MHC) class I antigens are covalently bonded at the C-terminal end to specific carrier molecules.

4. Antigenic constructs as claimed in claim 1, 2 or 3, in which one MHC class I antigen is covalently bonded to one specific carrier molecule in each case.

5. Antigenic constructs as claimed in claim 1, 2, 3 or 4, in which the specific carrier molecules are CD4 domains.

6. Antigenic constructs as claimed in claim 1, 2, 3 or 4, in which the specific carrier molecules are monoclonal antibodies.

7. Antigenic constructs as claimed in claim 6, in which the monoclonal antibodies are shortened in the constant part of the heavy chain.

8. Antigenic constructs as claimed in claim 1, 2, 3, 4, 5, 6 or 7, in which the MHC class I antigen is HLA B27w or HLA B27k.

9. Antigenic constructs as claimed in claim 1, 2, 3, 4, 5, 6, 7 or 8, which are prepared by genetic manipulation by fusion of the DNAs coding for them.

10. A process for the preparation of antigenic constructs as claimed in claim 1, 2, 3, 4, 5, 6, 7 or 8, which comprises the required parts of genes being fused in the form of their DNA, being provided with suitable regulation sequences and being expressed in suitable expression systems.

11. The use of the antigenic constructs as claimed in any one of claims 1 to 9 for the preparation of a pharmaceutical for the allogenization of target cells.

12. A pharmaceutical which contains antigenic constructs as claimed in any one of claims 1 to 9.

## Claims (Claims for the following Contracting State(s): ES, GR)

1. A process for the preparation of antigenic constructs from major histocompatibility complex (MHC) class I antigens and specific carrier molecules, which comprises the required parts of genes being fused in the form of their DNA, being provided with suitable regulation sequences and being expressed in suitable expression systems.

2. The process as claimed in claim 1, wherein the required parts of genes are ligated at the 3' end of the MHC class I gene.

3. The process as claimed in claim 1, wherein the required parts of genes are ligated at the 5' end of the MHC class I gene.

4. The process as claimed in claim 1, wherein one MHC class I antigen is linked to one specific carrier molecule in each case.

5. The process as claimed in claim 1, 2, 3 or 4, wherein CD4 domains are employed as specific carrier molecule.

6. The process as claimed in claim 1, 2, 3 or 4, wherein monoclonal antibodies are employed as specific carrier molecule.

7. The process as claimed in claim 1, 2, 3, 4, 5 or 6, wherein the HLA B27w of HLA B27k gene or molecule is employed.

8. The process as claimed in claim 6, wherein the monoclonal antibodies employed are shortened in the constant part of the heavy chain.

9. A process for the preparation of pharmaceuticals, which comprises mixing antigenic constructs prepared as claimed in claim 1, 2, 3, 4, 5, 6, 7 or 8 with conventional carrier materials.

## Revendications (Revendications pour l'(les) Etat(s) contractant(s) suivant(s): AT, BE, CH, DE, FR, GB, IT, LI, LU, NL, SE)

1. Constructions antigéniques caractérisées en ce que des antigènes de la classe I du complexe majeur d'histocompatibilité (CMH) (*Major Histocompatibility Complex*) sont liés par covalence à l'extrémité C-terminale ou N-terminale avec des molécules de support spécifiques.

2. Constructions antigéniques selon la revendication 1, caractérisées en ce que des antigènes de la classe I du complexe majeur d'histocompatibilité (CMH) sont liés par covalence à l'extrémité amino-terminale avec des molécules de support spécifiques.

3. Constructions antigéniques selon la revendication 1, caractérisées en ce que des antigènes de la classe I du complexe majeur d'histocompatibilité (CMH) sont liés par covalence à l'extrémité C-terminale avec des molécules de support spécifiques.

4. Constructions antigéniques selon la revendication 1, 2 ou 3, caractérisées en ce qu'un antigène de la classe I du CMH est lié par covalence respectivement à une molécule de support spécifique.

5. Constructions antigéniques selon la revendication 1, 2, 3 ou 4, caractérisées en ce que les molécules de support spécifiques sont des domaines CD4.

6. Constructions antigéniques salon la revendication 1, 2, 3 ou 4, caractérisées en ce que les molécules de support spécifiques sont des anticorps monoclonaux.

7. Constructions antigéniques selon la revendication 6, caractérisées en ce que les anticorps monoclonaux sont raccourcis dans la partie constante de la chaîne lourde.

8. Constructions antigéniques selon la revendication 1, 2, 3, 4, 5, 6 ou 7, caractérisées en ce que l'antigène de la classe I du CMH est HLA B27w ou HLA B27k.

9. Constructions antigéniques selon la revendication 1, 2, 3, 4, 5, 6, 7 ou 8, caractérisées en ce qu'on les prépare par génie génétique grâce à la fusion des ADN codant pour des constructions.

10. Procédé de préparation de constructions antigéniques selon la revendication 1, 2, 3, 4, 5, 6, 7 ou 8, caractérisé en ce que les fragments de gènes nécessaires, sous forme de leur ADN fusionné, sont pourvus de séquences de régulation appropriées et exprimés dans des systèmes d'expression appropriés.

11. Utilisation des constructions antigéniques selon l'une des revendications 1 à 9 pour la préparation d'un médicament pour l'allogénisation de cellules cibles.

12. Médicaments contenant les constructions antigéniques selon l'une des revendications 1 à 9.

## Revendications (Revendications pour l'(les) Etat(s) contractant(s) suivant(s): ES, GR)

1. Procédé de préparation de constructions antigéniques formées d'antigènes de la classe I du complexe majeur d'histocompatibilité (CMH) (*Major Histocompatibility Complex*) et de molécules de support spécifiques, caractérisé en ce que les fragments de gènes nécessaires, sous forme dc leur ADN fusionné, sont pourvus de séquences de régulation appropriées et exprimés dans des systèmes d'expression appropriés.

2. Procédé selon la revendication 1, caractérisé en ce que les fragments de gènes nécessaires sont liés à l'extrémité 3' du gène de la classe I du CMH.

3. Procédé selon la revendication 1, caractérisé en ce que les fragments de gènes nécessaires sont liés à l'extrémité 5' du gène de la classe I du CMH.

4. Procédé selon la revendication 1, caractérisé en ce qu'un antigène de la classe I du CMH est lié respectivement à une molécule de support spécifique.

5. Procédé selon la revendication 1, 2, 3 ou 4, caractérisé en ce qu'on utilise des domaines CD4 comme molécule de support spécifique.

6. Procédé selon la revendication 1, 2, 3 ou 4, caractérisé en ce qu'on utilise des anticorps monoclonaux comme molécule de support spécifique.

7. Procédé selon la revendication 1, 2, 3, 4, 5 ou 6, caractérisé en ce qu'on met en oeuvre le gène ou la molécule de HLA B27w ou de HLA B27k.

8. Procédé selon la revendication 6, caractérisé en ce que les anticorps monoclonaux utilisés sont raccourcis dans la partie constante de la chaîne lourde.

9. Procédé de préparation de médicaments, caractérisé en ce qu'on mélange des constructions antigéniques préparées selon la revendication 1, 2, 3, 4, 5, 6, 7 ou 8 avec des matériaux de support usuels.
